Europäisches Patentamt

European Patent Office

Office européen des brevets

(19)

(11) Numéro de publication: **0 029 787**
**B1**

(12) **FASCICULE DE BREVET EUROPEEN**

(45) Date de publication du fascicule du brevet:
11.09.85

(21) Numéro de dépôt: **80401673.1**

(22) Date de dépôt: **21.11.80**

(51) Int. Cl.⁴: **A 61 F 2/02**

(54) **Procédé de réalisation d'artifices utilisables in-vivo et artifices réalisés par ce procédé.**

(30) Priorité: **26.11.79 FR 7929038**

(43) Date de publication de la demande:
**03.06.81 Bulletin 81/22**

(45) Mention de la délivrance du brevet:
**11.09.85 Bulletin 85/37**

(84) Etats contractants désignés:
**AT BE CH DE FR GB IT LI LU NL SE**

(56) Documents cités:
**CH - A - 563 152**
**DE - A - 2 313 678**
**DE - A - 2 717 615**
**FR - A - 1 571 184**
**FR - A - 2 277 792**
**FR - A - 2 282 852**
**FR - A - 2 318 617**
**FR - A - 2 336 913**
**FR - A - 2 383 656**
**FR - A - 2 391 175**
**FR - A - 2 421 595**
**GB - A - 2 010 122**

(73) Titulaire: **Davidas, Jean-Paul, 110, Rue Lamarck, F-75018 Paris (FR)**

(72) Inventeur: **Davidas, Jean-Paul, 110, Rue Lamarck, F-75018 Paris (FR)**

(74) Mandataire: **Laget, Jean-Loup et al, Cabinet Pierre Loyer 18, Rue de Mogador, F-75009 Paris (FR)**

BUNDESDRUCKEREI BERLIN

# Description

L'invention se rapporte aux artifices utilisables in-vivo en chirurgie générale ou buccale et à un procédé de réalisation de ces artifices.

Par artifices on entend des corps ou éléments essentiellement métalliques susceptibles d'être utilisés en chirurgie pour constituer une prothèse intra-osseuse ou juxta-osseuse ou pour servir à fixer une prothèse buccale par exemple. L'invention concerne donc la chirurgie générale (ostéosynthèse) et la chirurgie buccale (implantologie).

L'utilisation des artifices métalliques notamment en implantologie est assez répandue, en raison de l'efficacité et de la qualité des fixations qu'ils procurent aux prothèses en particulier. Un des avantages de ces artifices métalliques est de n'entraîner qu'une mutilation négligeable par rapport à la grande surface de contact avec les tissus de support qu'autorise leur conception.

L'utilisation des artifices métalliques, en métal pur ou en alliage, est cependant critiquée en raison du rapport permanent et intime qui s'établit entre le métal ou l'alliage de l'artifice et le milieu biologique récepteur.

On a essayé d'assurer à des artifices métalliques une biocompatibilité à l'égard des tissus et du milieu qui les reçoivent, en recouvrant les artifices d'une couche de revêtement en verre ou en céramique par exemple. Les couches de revêtement que l'on est arrivé à faire ont une épaisseur relativement importante, de l'ordre du millimètre. Dans certains cas on est même arrivé à faire des couches plus minces, dont l'épaisseur peut descendre jusqu'au micron.

Le FR-A-2 277 792 décrit un article de prothèse dentaire recouvert d'une première couche d'oxyde métallique et d'une deuxième couche biocompatible en porcelaine non déformable.

Le DE-A-2 717 615 décrit une embase rigide pour prothèse dentaire recouverte d'une couche d'oxyde métallique très dur et non déformable.

Les FR-A-2 383 656, FR-A-2 336 913 et FR-A-2 318 617 décrivent des supports de prothèse métalliques, portant deux couches de revêtement non déformables, d'épaisseur notable.

Le FR-A-1 571 184 est relatif à une prothèse revêtue d'une couche de carbone pyrolytique, cristalline et d'épaisseur au moins égale à 50 μ.

Il se trouve, cependant que ces couches de revêtement d'une épaisseur égale ou supérieure au micron correspondent toutes à une structure de couche cristalline. Aussi, lorsque le praticien met en forme, on déforme en les mettant en place, les artifices recouverts d'une telle couche, des fissurations se produisent. l'isolement prévu par la disposition de la couche de revêtement n'est alors pas assuré, non plus que las biocompatibilité à l'égard des tissus recevant l'artifice.

L'invention a pour but d'éviter les inconvénients de ces couches de revêtement non déformables, et d'assurer à des artifices métalliques une biocompatibilité à l'égard des tissus qui les reçoivent.

L'invention a pour objet un procédé de réalisation d'artifices utilisables en-vivo, du type dans lequel une armature métallique est recouverte d'une couche de revêtement d'un composé biocompatible isolant, réfractaire, amorphe et non poreux, caractérisé en ce que ladit couche est appliquée de telle sort que son épaisseur soit comprise entre 100 A° et 0,5 μ et qu'ainsi cette couche soit déformable.

Selon l'invention, l'application de la couche mince est assurée par pulvérisation cathodique, réactive ou à radiofréquence, par exemple.

Selon l'invention également, le composé isolant biocompatible est pris dans le groupe comprenant l'alumine, les verres, les dérivés de la silice ou du quartz, les apatites et leurs dérivés, les oxydes, les nitrures et les sulfures d'aluminium, et les carbones.

L'invention a également pour objet un artifice utilisable invivo, du type dans lequel une armature métallique est recouverte d'une couche de revêtement d'un composé biocompatible, isolant, réfractaire, amorphe et non poreux, caractérisé en ce que la couche de revêtement est déformable et d'épaisseur comprise entre 100 A° et 0,5 μ, de façon à supporter sans fissuration la mise en forme et les déformations de l'artifice.

L'invention s'applique particulièrement à la réalisation des artifices utilisables en chirurgie osseuse et en chirurgie buccale. A titre d'exemple, un implant dentaire préfabriqué est mis en forme par le praticien au moment de sa mise en place. Au cours de cette mise en forme, des déformations et contraintes mécaniques sont appliquées à l'implant. Si un tel implant a préalablement été isolé par une couche de céramique par exemple d'une épaisseur supérieure à 1 μ, la mise en forme risque d'altérer la céramique, et la biocompatibilité de l'implant n'est plus assurée.

Selon l'invention, au contraire, une couche de revêtement d'épaisseur comprise entre 100 A° et 0,5 μ reste déformable sans fissuration et ne présente pas ce risque d'altération.

Il en résulte que l'artifice mis en place, constitué d'une armature métallique préfabriquée et d'une couche de revêtement biocompatible, présente toutes les garanties souhaitées de sécurité et de biocompatibilité.

La couche mince de revêtement a une épaisseur comprise entre eviron 100 Angströms et environ 0,5 μ. Elle est appliquée par exemple par pulvérisation cathodique réactive (bombardement d'une cible par un gaz réactif sous forme de plasma), ou par pulvérisation cathodique à radiofréquence.

Au cours de la phase d'application de la couche de revêtement sur l'artifice, il est nécessaire d'utiliser un vide poussé afin d'éviter le dépôt d'impuretés qui affecteraient le caractère isolant de la couche de revêtement. Il est en effet indispensable que cette couche soit isolante, non poreuse, et de structure amorphe. Elle est alors non biodégradable et joue parfaitement son rôle.

L'originalité du procédé selon l'invention consite à utiliser les propriétés et avantages liés à la nature métallique des implants tout en assurant par la couche mince de revêtement, la biocompatibilité des artifices réalisés à l'égard des tissus destinés à les recevoir.

Avec ce procédé peut être évité le risque de détérioration de la couche de revêtement au cours de la mise en forme de l'implant ou de sa mise en place.

Les caractéristiques mécaniques et physiques des implants métalliques et des revêtements proposés présentent aussi l'avantage d'offrir une sécurité dans l'avenir de leur liaison réciproque.

L'invention a été décrite dans l'exemple de réalisation d'implants dentaires, mais elle s'applique également aux artifices utilisés en chirurgie osseuse, qu'il s'agisse d'éléments de prothèse ou d'accessoires de fixation par exemple, notamment de fils et plaques d'ostéo-synthèse.

Le procédé selon l'invention permet d'élaborer sur mesure les artifices métalliques, dans la mesure où ils sont faciles à couler, car la couche protectrice les isole du milieu biologique et empêche les phénomènes d'oxydation et de corrosion des métaux ou alliages constituant l'artifice.

## Revendications

1. Procédé de réalisation d'artifices utilisables in-vivo, du type dans lequel une armature métallique est recouverte d'une couche de revêtement d'un composé biocompatible, isolant, réfractaire, amorphe et non poreux, caractérisé en ce que ladite couche de revêtement est appliquée de telle sorte que son épaisseur soit comprise entre 100 A° et 0,5 μ et qu'ainsi cette couche soit déformable.

2. Procédé selon la revendication 1, caractérisé en ce que le composé isolant biocompatible est pris dans le groupe comprenant l'alumine, les verres, les dérivés de la silice ou du quartz, les apatites et leurs dérivés, les oxydes, les nitrures et les sulfures d'aluminium et les carbones.

3. Procédé selon l'ensemble des revendications 1 et 2, caractérisé en ce que l'application de la couche de revêtement est assurée par pulvérisation cathodique, réactive ou à radiofréquence.

4. Artifice utilisable in-vivo, du type dans lequel une armature métallique est recouverte d'une couche de revêtement d'un composé biocompatible, isolant, réfractaire, amorphe et non-poreux, caractérisé en ce que la couche de revêtement est déformable et d'épaisseur comprise entre 100 A° et 0,5 μ, de façon à supporter sans fissuration la mise en forme et les déformations de l'artifice.

## Patentansprüche

1. Verfahren zur Herstellung in vivo verwendbarer Ersatzglieder bzw. -teile des Typs, bei dem eine metallische Verstärkung bzw. Einlage mit einer Überzugsschicht aus einer biologisch verträglichen, isolierenden, feuerfesten, amorphen und nicht porösen Verbindung überzogen ist, dadurch gekennzeichnet, daß die erwähnte Überzugsschicht derart aufgebracht wird, daß ihre Dicke zwischen 10,0 nm und 0,5 μm liegt, und daß diese Schicht folglich verformbar ist.

2. Verfahren nach Anspruch 1, dadurch gekennzeichnet, daß die isolierende, biologisch verträgliche Verbindung aus der Gruppe, die Aluminiumoxid, die Gläser, die Derivate von Siliciumdioxid oder Quarz, die Apatite und ihre Derivate, die Oxide, die Nitride und die Sulfide von Aluminium und die Kohlenstoffe umfaßt, ausgewählt wird.

3. Verfahren nach Ansprüchen 1 und 2, dadurch gekennzeichnet, daß die Überzugsschicht durch reaktive oder durch Hochfrequenz-Kathodenzerstäubung aufgebracht wird.

4. In vivo verwendbares Ersatzglied bzw. -teil des Typs, bei dem eine metallische Verstärkung bzw. Einlage mit einer Überzugsschicht aus einer biologisch verträglichen, isolierenden, feuerfesten, amorphen und nicht porösen Verbindung überzogen ist, dadurch gekennzeichnet, daß die Überzugsschicht verformbar ist und eine Dicke hat, die zwischen 10,0 nm und 0,5 μm liegt, so daß die Überzugsschicht dem Gestalten und den Verformungen des Ersatzgliedes bzw. -teils standhält, ohne rissig zu werden.

## Claims

1. Method for producing artificial aids suitable for use in vivo, of the type wherein a metal frame is covered with a covering layer of a non-porous, amorphous, refractory, insulating and biocompatible compound, characterised in that the said covering layer is applied in such a manner that its thickness is comprised between 100 A° and 0.5 μ and that therefore the said layer is deformable.

2. Method according to claim 1, characterised in that the insulating biocompatible compound is taken from the group comprising alumina, glasses, derivatives of silica or quartz, apatites and derivatives thereof, oxides, nitrides and sulphides of aluminium, and carbons.

3. Method according to claims 1 and 2, characterised in that the application of the covering layer is effected by reactive, radio-frequency or cathodic atomisation.

4. Artificial aid usable in vivo, of the type wherein a metal frame is covered with a covering layer of a nonporous, amorphous, refractory, insulating, biocompatible compound, characterised in that the covering layer is deformable and of a thickness comprised between 100 A° and 0.5 μ, in such a manner as to withstand without cracking the shaping of the aid and the deformations which it undergoes.